# EUROPEAN PATENT APPLICATION

(11) **EP 4 483 720 A1**
(43) Date of publication of application: **01.01.2025**
(21) Application number: 23831981.8
(22) Date of filing: 26.04.2023
(51) Int. Cl.: A23J 1/18, C12N 1/16, A23J 3/20, A23L 21/15, C12R 1/645

(54) **<SMALLCAPS/>? ? ?KLUYVEROMYCES MARXIANUS? ? ? ? ?PRODUCTION PROCESS AND FORMULATION OF A SINGLE-CELL PROTEIN BY FERMENTING THE YEAST**

(30) Priority: 30.06.2022 MX 2022008248
(71) Applicant: Proteo Alimentaria, S.A.P.I de C.V., Ciudad de Mexico, 05348 (MX)
(72) Inventor: NAVARRO LIZÁRRAGA, Miguel Ángel, COLIMA, 20020 (MX)
(74) Representative: Carlos Hernando, Borja
(86) International application number: PCT/MX2023/050025
(87) International publication number: WO 2024/005625

(57) **Abstract**

The present invention relates to a novel fermentation process using a particular strain of *Kluyveromyces marxianus,* referred to as KMPROTEO 1, to obtain a single-cell protein useful in the food industry.

In the present description, the invention discloses a process for fermenting dextrose and other consumables to obtain a single-cell biomass. The disclosed process enables a high-protein product to be obtained that is in the range of 50 to 60% for use in the food industry. The disclosed process uses dextrose as a propagation medium for use by *Kluyveromyces marxianus* to obtain a single-cell biomass and, subsequently, a single-cell protein.

## Description

### OBJECT OF THE INVENTION

The present invention relates to a novel fermentation process using a particular strain of *Kluyveromyces marxianus,* referred to as KMPROTEO 1, to obtain a single-cell protein useful in the food industry.

The strain *Kluyveromyces marxianus,* referred to as KMPROTEO 1, has been deposited under the terms of the Budapest Treaty on the International Recognition of the Deposit of Microorganisms for the Purposes of Patent Procedure, in the *Centro National de Recursos Genéticos* (CNRG) (National Center for Genetic Resources) of the *Instituto National de Investigaciones Forestales, Agricolas y Pecuarias* (National Institute for Forestry, Agricultural and Livestock Research), which was assigned accession number CM-CNRG TB66 after the corresponding certification of viability.

### BACKGROUND

Proteins are essential nutrients for the processes and maintenance of cellular systems in the human body. Currently, the major protein sources in food for human consumption are of animal and plant origin. Proteins are one of three essential macronutrients, the other two being carbohydrates and lipids, all of which are integral to the maintenance of the human body.

Due to the great variability among proteins, there are several ways to classify them. One of the most commonly used classifications depends on the solubility of proteins, in which 5 groups are identified:
1. Albumins, which are soluble in water and dilutable in saline solutions and, furthermore, do not have a defined amino acid conformation;
2. Globulins, which are slightly soluble in water but highly soluble in saline solutions and, furthermore, are rich in glutamates and aspartates;
3. Histones, which are soluble in acidic saline solutions and rich in basic amino acids;
4. Prolamins, which are insoluble in water but soluble in ethanol-water mixtures; and
5. Scleroproteins, which are insoluble in water and saline solutions and include fibrous proteins rich in glycyl, prolyl, and alanyl.

Proteins can also be classified, according to their nutritional value, into complete proteins, which are those with an essential amino acid profile, and incomplete proteins, which are deficient in one or more essential amino acids. Most proteins of animal origin are complete proteins, and proteins of plant origin are mostly incomplete proteins.

Current techniques to increase protein production are based on scientific and technological techniques available for agriculture and food processing industries. One proposed solution to prevent the shortage of protein sources is to improve agro-industrial processes to obtain incremental improvements; however, this approach is increasingly more expensive. A second option to increase protein supply is to develop new sources for obtaining them, using biotechnological processes, converting secondary sources or industrial wastes into high quality protein for human consumption.

The classification of the ways of obtaining sustainable protein identifies three groups: traditional, non-traditional, and innovative sources.

Traditional sources come from agriculture and livestock. Among these, the harvesting of plants, seeds, nuts, and other agricultural products can be highlighted. This category also includes livestock and animal husbandry, both for the meat and for the dairy products derived from it. Also included in this classification is aquaculture, i.e., those that come from farmed fish, shellfish, and mollusks.

Non-traditional sources include by-products with high protein content from the dairy industry and mills. Also included in this source are products from the sea, plant biomass, such as stems or roots, microorganisms, or exotic nuts. Moreover, this category includes animal blood, viscera, and organs, which are not commonly consumed in some developed countries. Finally, this item includes natural flora and fauna not found in captivity.

Among innovative sources, proteins obtained by fermenting microorganisms, among which yeasts, microalgae, and cyanobacteria stand out, can be mentioned. Also included are proteins derived from insects and proteins obtained by chemical and laboratory synthesis. Each of these innovative sources includes technical risks and problems to be solved in order to incorporate said proteins into finished food products.

The presentation of the protein can be as varied as the sources from which it is obtained. The simplest way is to simply grind and dry the original protein source; however, the content and bioavailability of the protein is low. The next step is to concentrate the protein, for which there are various methods in the industry. This form of protein has the following advantages:
1. The ingredient has a low relative moisture and can be stored in a simpler manner and for a longer time;
2. Virtually most antinutrients and toxins are taken out of the product, such that they reach a permissible range in food for human consumption;
3. The concentration of protein is optimal, and its application in food is done in a simpler manner.

The most important nutritional factors for classifying proteins are amino acid composition and bioavailability.

Amino acid composition refers primarily to whether the protein is complete or incomplete. In addition to this, the amino acid profile is focused on covering the minimum requirements of a child of preschool age. For this purpose, the "Protein Digestibility Corrected Amino Acid Score" method, developed by FAO/WHO, was created, and this method measures the content of the first limiting amino acid of the analyzed protein as a percentage of the content of that amino acid in a known reference standard.

Proteins added as an ingredient to a matrix must also meet consumer acceptance factors, such as texture, flavor, and appearance in the finished product.

In recent years, plant proteins have positioned themselves as protein sources that can replace animal sources. At the industrial level, the main sources are legumes, such as peas, soybeans, beans, or rice. However, these proteins are incomplete proteins, usually due to the lack of one or more of the essential amino acids. A solution to this is to mix proteins from different legumes, for example, protein from beans, peas, and rice. This makes processing and incorporation into food complicated due to the different flavors and aftertaste of the different legumes.

On the one hand, crops of this type have the advantage of having an almost assured supply, since the agricultural industry produces them in large quantities, in addition to the fact that these crops are usually homogeneous, so this raw material has a well-defined standard, which greatly helps in the extraction process.

On the other hand, one of the drawbacks of using this protein source is its relatively high cost and the low possibility of achieving significant improvements to considerably reduce its cost. In other words, since legumes are products of the agricultural industry, the problem is that this industry has already reached a maximum efficiency point at which it is difficult to obtain improvements that would significantly reduce costs. Since legumes are a field product, they are susceptible to weather conditions, so in the case of a climatic event or any other type of event affecting the crop, the supply of this protein source will be affected. Likewise, the price of the product is highly volatile and varies significantly between crop cycles. Another important drawback is that when using legumes as a source for obtaining proteins, as a result of the behavior and performance of the market, when the demand for a certain crop increases, the price of the seed will necessarily increase.

Proteins from non-conventional sources, including insects, have considerable advantages and drawbacks. Among the advantages is their ease of growth, as they do not require strict conditions for reproduction and can be produced in closed environments where their development is closely monitored. Insects require less than half the feed required by farm animals to produce the same amount of biomass. This source is high in protein and has a good amino acid profile. Its main drawback at present is the scalability and replicability of the production process. The insects currently consumed in several parts of the world are trapped rather than farmed; that is, their supply is small and highly scattered according to geographical and cultural conditions. Insect farming is a relatively new and uncommon practice. This means that this industry has not yet reached the level of efficiency that exists in agriculture or livestock farming. Another problem is control in the production process, where, if the harmlessness of the process is not ensured, there can be a significant source of contamination in the finished product. This is common in insect growing practices of this type, where standardized and predefined quality standards are not followed. The aspect of consumer acceptance of products of this type, as well as the regulations, which in many cases are not very clear with respect to this protein source, must also be assessed.

Synthetic protein sources obtained at the laboratory level simply do not have the necessary scalability or economic efficiency to be launched on the market. These protein sources are very expensive to produce, in addition to having a negative acceptance by consumers.

One of the alternatives to solve this problem is microorganisms, which can meet some of the world's food needs. Possibly the most important potential use of microorganisms is not as a complete diet, but as a vitamin and protein supplement.

It is in this context where the sources for obtaining protein by fermenting microorganisms are envisioned as a viable, economical, safe, replicable, and scalable option. The specific case of the invention disclosed in the present description relates specifically to a novel fermentation process of a particular strain of *Kluyveromyces marxianus,* referred to as KMPROTEO 1, to subsequently extract its protein phase, with a final protein content in dry weight greater than 50%.

The industrial fermentation industry is one of the agro-industries that has shown the greatest growth and potential in the last 20 years, generating large-scale products for industries such as food, nutraceuticals, cosmetics, and biopharmaceuticals, among others. Among the main products are yeast extracts and specialty products, produced mainly by companies that sell these products to the bakery and brewery sectors. In recent years, these companies are diversifying into other markets, including single-cell protein production.

Referring to the protein concentration, yeast contains about 40% protein. A part of this protein is found in its cell interior and another part is found in its cell wall. It is for this reason that any method of extraction and purification of this protein must include a component of extraction of proteins in the cell wall.

By giving such a specific use to yeast as a source for obtaining proteins, the following advantages can be seen:
1. The protein obtained can be inexpensive, as long as the process is simple, replicable, and scalable;
2. Protein is generated from a clean process, trying to take maximum advantage of residual by-products;
3. It is possible to obtain a constant supply of raw materials, since they are available all year round, without depending on weather conditions;
4. Protein production and batches can be planned at any time of the year and in any climate, considering that production is carried out in a production plant with bioprocess equipment that does not come into contact with the environment;
5. The resulting protein is a complete protein, i.e., it has an optimal amino acid profile for human consumption;
6. The dextrose used as a raw material is a homogeneous source, thus facilitating the fermentation and extraction process;
7. Production will not be affected by weather factors, as occurs with an agricultural product;
8. The process is replicable, as there are fermentation companies operating in virtually every country in the world.

Currently, there is yeast protein that is sold commercially; however, said protein has a brown color as well as a flavor and smell similar to yeast, characteristics that are not pleasant for the consumer.

In view of the above, the alternative of changing the mode of production, using biotechnological processes, in order to thereby produce abundant, highly nutritious food of excellent quality and low price, is appealing and viable.

The traditional mode of production does not help the purpose of feeding humanity and will continue to be the main global pollution problem, since a large part of the food industry generates waste that contains a high level of carbohydrates, and said organic waste is dumped into the sewage system, causing intense environmental pollution. Such waste further generates unpleasant smells, causes serious diseases, and damages soil fertility and water quality.

There are multiple examples of fermentation processes, the purpose of which is the production of cellular protein, for the purpose of (also) providing nutritional components such as proteins, nucleic acids, polysaccharides, vitamins, and the like, which are used as food supplements, used in livestock feed, or for the production of food supplements for human consumption.

For example, international patent application WO 2013/156703, entitled "Baked goods containing *Kluyveromyces marxianus* or *Kluyveromyces lactis*"*,* describes an invention consisting of baked goods containing yeasts not intended for baking. The invention relates to the use of nonconventional yeasts for baking baked goods having a specific volume greater than 3 mL/g; the yeasts used impart special aromatic notes to the goods containing same. The document also describes a method for preparing baked goods.

International patent application WO2017/036294, entitled *"Kluyveromyces marxianus* and use thereof", describes a particular strain of *Kluyveromyces marxianus,* with biological material deposit number CGMCC No.10621; the use of this strain of *Kluyveromyces marxianus* which comprises constructing a recombinant protein expression system and fermentation; and an expression vector used for *Kluyveromyces marxianus,* an enzyme/a protein expressed by the yeast and the uses of any of those materials mentioned above in the fields of feed, feed additives, food, vaccines, drugs, and the like. Furthermore, the aforementioned *Kluyveromyces marxianus* can provide the nutritional components of proteins, nucleic acids, polysaccharides and vitamins, and the like, which can complement the nutritional components needed during the growth of animals, and can help animals with oxidation and the metabolism of lactose and lipids.

Korean patent 1010803780000, entitled "Compositions and health food containing *Kluyveromyces marxianus* for prevention and treatment of allergies, inflammation, and asthma", discloses a composition containing *Kluyveromyces marxianus* to suppress cytokines (IL-4, IL-5, and IL-13) and to suppress prostaglandins and leukotrienes. The document describes a composition for the prevention and treatment of allergies, inflammation, and asthma containing *Kluyveromyces marxianus* as the active ingredient. The composition is used by administering same to a patient as a pharmaceutically effective ingredient for the prevention and treatment of allergies, inflammation, and asthma. It also describes a health food containing *Kluyveromyces marxianus* as the active ingredient.

Chinese patent application 202011037696.7 describes a preparation method for preparing active dry yeast of *Kluyveromyces marxianus.* The described invention belongs to the technical field of live microbial preparations and, in particular, relates to a preparation method for preparing active dry yeast of *Kluyveromyces marxianus.* The preparation method comprises the following steps: filtering a fermentation liquid of *Kluyveromyces marxianus* to obtain a fresh yeast paste; redissolving the fresh yeast paste in a solution of pretreatment agent to obtain a suspension of redissolved cells and filtering to obtain the pretreated yeast paste; dispersing the pretreated yeast paste, adding a protective agent and continuing to stir for 20 to 30 minutes to obtain the pretreated yeast paste; and finally, extruding the pretreated yeast paste into wet yeast granules, adding to a fluidized bed dryer and drying in two stages, wherein in the first stage, drying is performed at 50 to 65°C for 5 to 15 minutes, and the water content of the material is controlled at 25 to 35% after drying is completed; and in the second stage, the drying temperature is lowered to 35-42°C until the water content of the material is 4-6%, and drying is completed to obtain the active dry yeast product of *Kluyveromyces marxianus.*

Japanese patent application 2009233970 relates to a method for producing breads containing whey powder and the breads obtained by the method, which is a method of preparing breads containing whey powder and the breads obtained by the method. Breads containing high quality whey powder can be produced by mixing cultured fungus bodies of the lactose-fermenting yeast of *Kluyveromyces marxianus,* having the high bread dough-fermenting power, with the normal baker's yeast of *Saccharomyces cerevisiae.*

Bulgarian patent application 8705489, entitled "Method for preparing protein", relates to a method applicable in the meat processing, dairy, and sugar industries, and in catering for the manufacture of products of increased biological and nutritional value. The method allows the use of total integrated milk whey from cheese production. According to the described method, milk whey from cheese production is deproteinized by means of ultrafiltration at a temperature between 40 and 43°C, an inlet pressure of 0.67 MPa, an outlet pressure of 0.60 MPa, and a GR 61 P membrane, and then, the resulting ultrafiltrate containing 4.5-4.8% lactose is supplemented with 0.5-0.9% (NH₄)₂HPO₄ and 10 to 20% corn extract, as a percentage of the ultrafiltrate, the pH is brought to 5.0, inoculated with a 24-hour culture of a microorganism of the strain *Kluyveromyces marxianus var. lactis,* registered in the NBPMCC under number 1502 in an inoculum size of 8 to 12% and subsequently cultured with aeration at a rate of 0.008 s^{{-1}}, at 52.3 rad/s and a temperature of 28 to 32°C.

Canadian patent application 2799452 relates to a "Fermentation process of a substrate using a mixed culture for the production of an edible biomass for animal and/or human consumption". The invention is directed to processes, combinations, uses and biomass comprising a combination of yeasts and bacterial strains, for the production of consumable biomass from substrates comprising a simple sugar. More particularly, the claimed subject matter includes the use of *Lactobacillus fermentum, Kluyveromyces marxianus,* and *Saccharomyces unisporus.*

International patent application WO 2017/023913, entitled "Food products comprising cell wall material", relates to food products comprising cell wall material, which provides food products having structures, textures, and other properties similar to those of animal meat, and that comprise substantial amounts of cell wall material. Also provided are methods and processes for producing such food products.

Australian patent application 2019408401, entitled "Functional yeast protein concentrate", relates to a method for the preparation of a yeast protein concentrate. Said method comprises the lysis of yeast cells in a suspension that is adjusted to a particular pH prior to lysis, subsequently subjecting the soluble fraction obtained from lysis to filtration to reduce the content of molecules smaller than 30 kDa, and optionally drying the solution obtained from filtration. The present invention further relates to a yeast protein concentrate obtainable by means of the described method. The yeast protein concentrate comprises a high amount of proteins which are still folded and are therefore capable of aggregation to form a solid protein matrix upon heating. In addition, the described yeast protein concentrate will be of unobtrusive taste and is therefore particularly suited for use in the preparation of food items, such as meat substitute products.

US patent 17049897, entitled "Yeast proteins", relates to a method for obtaining yeast proteins comprising the following steps: a) providing a yeast cream; b) exposing this yeast cream to a thermal plasmolysis at a temperature between 70 and 95°C for a period between 30 seconds and 4 hours, preferably between 1 minute and 3 hours, more preferably between 40 minutes and 2 hours; b) separating the insoluble fraction and the soluble fraction; c) subjecting the insoluble fraction to the activity of at least one ribonuclease and a glucanase, sequentially or simultaneously, at a temperature between 40 and 65°C, preferably 60°C, for a period between 8 and 24 hours, preferably 18 hours; d) separating the insoluble fraction from the soluble fraction; wherein the insoluble fraction collected in step d) has no taste, has a nucleotide content less than 3% and a true protein content of at least 72%. Step b) is optional. In this case, the entirety of the composition obtained after thermal plasmolysis of the yeast cream is subjected to enzymatic activity.

International patent application WO 2011/140649, entitled "Fermentation process of a substrate using a mixed culture for the production of an edible biomass for animal and/or human consumption", is directed to processes, combinations, uses and biomass comprising a combination of yeasts and bacterial strains, for the production of consumable biomass from substrates comprising a simple sugar. More particularly, the claimed subject matter includes the use of *Lactobacillus fermentum, Kluyveromyces marxianus* and *Saccharomyces unisporus.*

The paper by Marta Elena Cori de Mendoza, et al., "Obtención y caracterización de dos concentrados proteicos a partir de biomasa de Kluyveromyces marxianus var. Marxianus cultivada en suero lácteo desproteinizado" (Attainment and characterization of two protein concentrates from biomass of Kluyveromyces marxianus var. marxianus grown in deproteinized whey), Revista Cientifica vol. 16 No. 3, 2006, describes a process where deproteinized and supplemented whey is used to adjust lactose to 1.5% by supplementing with ammonium sulfate. The yeast used is *Kluyveromyces marxianus,* and fermentation is carried out using a silicone-based defoamer. The paper points out that numerous substrates have been studied for the production of single-cell biomass, whey being considered one of the most important due to its low price and availability, apart from the fact that it is usually an important contaminant in waste water, which causes serious problems in the operation of purification plants.

Therefore, it is proposed to use same as a fermentation substrate for microorganisms capable of assimilating lactose, such as the yeast *Kluyveromyces marxianus var. Marxianus,* in order to obtain microbial biomass, also known as "single-cell protein". This has a protein content that ranges between 40 and 80% on a dry basis and its quality is more similar to animal protein than to plant protein. However, the use of single-cell protein for human consumption has important limitations: the presence of cell walls, which reduce the bioavailability of proteins and contain antigenic and allergenic factors; the high content of nucleic acids (basically RNA), which can cause kidney disorders and gout; and finally, the reduced functional properties exhibited by dehydrated cell biomass. These limitations can be overcome by means of preparing protein concentrates, as described for *Candida tropicalis* and *Kluyveromyces marxianus var. Marxianus,* using alkaline extraction and isoelectric precipitation. However, this is not sufficient, given that the levels of nucleic acids must be reduced, either by chemical or enzymatic methods. Among chemical methods, one of the most accepted is phosphorylation with phosphorus oxychloride or alternatively with sodium trimetaphosphate, where protein concentrates have been obtained from *Saccharomyces cerevisiae,* which concentrates have then been dehydrated through sophisticated methods, such as lyophilization or spray drying, respectively. The results described in the paper demonstrate that a protein concentrate can be obtained from microbial biomass of *Kluyveromyces marxianus var. Marxianus* cultured in deproteinized whey. The yield values and cell concentration obtained were satisfactory for this microorganism.

The paper by Páez G., et al., "Perfil de aminoácidos de la proteina unicelular de Kluyveromuces marxianus var. Marxianus" (Amino acid profile of the single-cell protein of Kluyveromyces marxianus var. Marxianus), Interciencia Vol. 33 No. 44, 2008, describes that the yeasts of *Kluyveromyces, Candida, Schwanniomyces, Lipomyces, Pichia, Rhodotorula and Saccaromycopsis* are ideal for the production of single-cell protein (SCP). They are characterized by having a weak fermentative metabolism, having no glucose effect and growing on different substrates: molasses, whey (*Kluyveromyces*)*,* methanol (*Pichia*)*,* acid hydrolysate of sugar cane bagasse (*Saccharomyces cerevisiae* and *Candida utilis*)*,* starchy wastewater (*Shwanniomyces*)*,* sulfite liquor from the paper industry or n-alkanes (*Candida*)*.* The paper describes that when the substrate is whey and molasses, the yeast of choice is *Kluyveromuces marxianus var. Marxianus,* which is proven upon determining that the amino acid profile obtained in the aforementioned document shows a balanced distribution of their content in the single-cell protein of *K. marxianus,* when compared to international reference standards (FAO) and to egg protein and other conventional protein sources, suggesting the potential use of this single-cell protein (SCP) as a protein source.

The paper by Buitrago Glorymar, et al., 2008, "Continue production of single cell protein of Kluyveromyces marxianus var. Marxianus", Rev. Téc. Ing. Univ. Zulia, 31 (Especial Maracaibo), describes the effect of lactose concentration on the growth kinetics of *Kluyveromyces marxianus var. Marxianus* ATCC 8554 and the production of the enzyme b-D-galactosidase in previously deproteinized whey. The paper points out that the production of single-cell protein for human and animal consumption uses microorganisms such as *Saccharomyces cerevisiae, Candida utilis, Fusarium graminearum,* and *Kluyveromyces marxianus,* the latter being a microorganism that rapidly ferments lactose, a sugar that constitutes the essential part of the dry extract of whey at a concentration of 4 to 5% w/v. The study showed that biomass can be obtained from *K. marxianus,* at low lactose concentrations, with yields of 50%, which generates the possibility of establishing a system for continuous production of single-cell protein for use as a protein concentrate.

Compared to the described prior art, the method disclosed in the present description has the following advantages:
- The process disclosed in the present description uses raw materials that are a by-product of other industries, specifically dextrose obtained as a by-product from the production of industrial starches, thereby contributing to a circular economy;
- By using the method disclosed in the present description, fermenting biomass concentrations higher than 60 grams per liter are obtained in less than 20 hours, while other methods take between 30 and 60 hours to reach the same cell concentration;
- The process disclosed in the present description produces a protein of high biological value, increasing the biological value of the ingredient, because the enzymatic hydrolysis process of the present invention, together with cell autolysis, results in a highly bioavailable protein in one and the same unit operation;
- Unlike other processes, the process disclosed in the present description does not use environmentally harmful chemicals, such that the by-products obtained from the process described herein can be reincorporated into subsequent industrial processes, or even be used as crop enhancers in irrigation systems;
- The process disclosed in the present description generates a protein with a high biological value and with organoleptic characteristics widely sought after by the food industry, to be incorporated as an alternative protein source that contributes to improving the nutritional profile of the food to which it is added.

### BRIEF DESCRIPTION OF THE FIGURES

The novel aspects considered to be characteristic of the production process and formulation of the single-cell protein disclosed herein will be set forth with particularity in the attached claims. However, the invention itself, by its structural organization, in conjunction with other objects and advantages thereof, will be better understood in the following detailed description of certain preferred embodiments when read in connection with the attached figures, wherein:

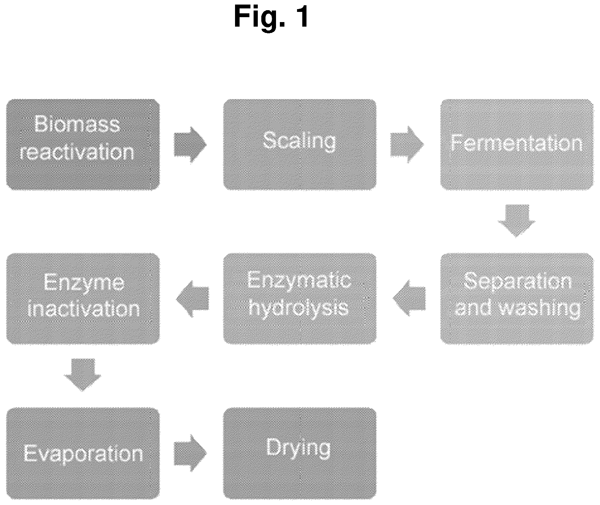

Figure 1 shows a flowchart describing the production process on a sequential basis of unit operations. The process starts with the reactivation of the strain, and then proceeds to a volumetric scaling, until reaching the final fermentation volume, where, once the fermentation is carried out, the biomass is separated, washed and subjected to enzymatic hydrolysis to ensure cell rupture. The enzymes used are subsequently inactivated, and the product is concentrated by evaporation and dried in a spray dryer.

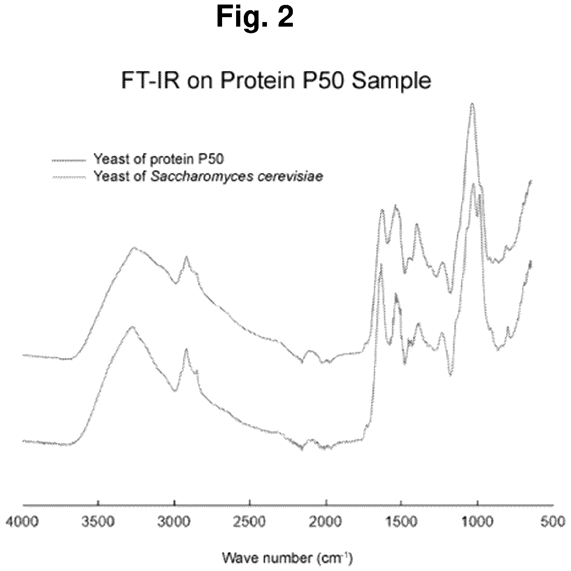

Figure 2 shows an infrared spectroscopy characterization of a sample of the yeast *Kluyveromyces marxianus,* compared with the yeast *Saccharomyces cerevisiae.* In the image, it can be seen with the naked eye how the absorbance curve of the yeast *Kluyveromyces marxianus* shows a higher absorbance range compared to the yeast *Saccharomyces cerevisiae.*

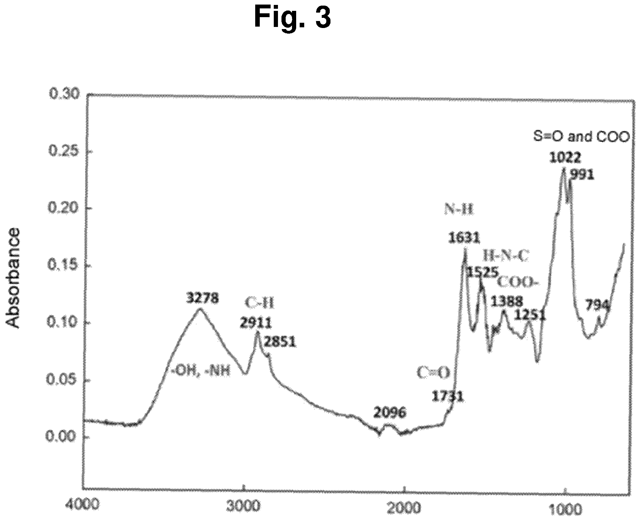

Figure 3 illustrates the types of majority bonds in the protein obtained with the process disclosed in the present invention. At a wavelength of 3,278 cm-1, it can be seen that most of the bonds are of the -OH and -NH type. At a wavelength of 2,911 cm-1, most of the bonds are of the C-H type. At a wavelength of 1,631 cm-1, most of the bonds are of the N-H type. At a wavelength of 1,525 cm-1, most of the bonds are of the H-N-C type. At a wavelength of 1,388 cm-1, most bonds are of the COO- type. At a wavelength of 1,022 cm-1, most of the bonds are of the S=O and COO type.

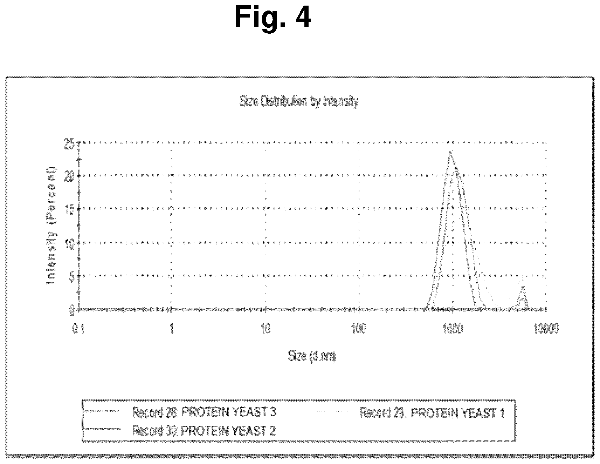

Figure 4 shows a particle size intensity dispersion, which was performed under the following conditions: For the determination of size, polydispersion index, and zeta potential, a 1:100 dilution of the particles with ultrapure water was performed by means of dynamic light scattering with Zeta Sizer N90 equipment (Malvern Instruments, Malvern, GB). The evaluation was performed at 25°C; for the measurement of size and polydispersity index (PDI) the refractive index of sunflower oil (1.43) was used.
   Several repetitions were carried out to determine the particle size of the sample, from which the illustrated graph was derived. Each color represents a repetition. Each peak represents a population, which the apparatus plots in signal intensity or population volume.

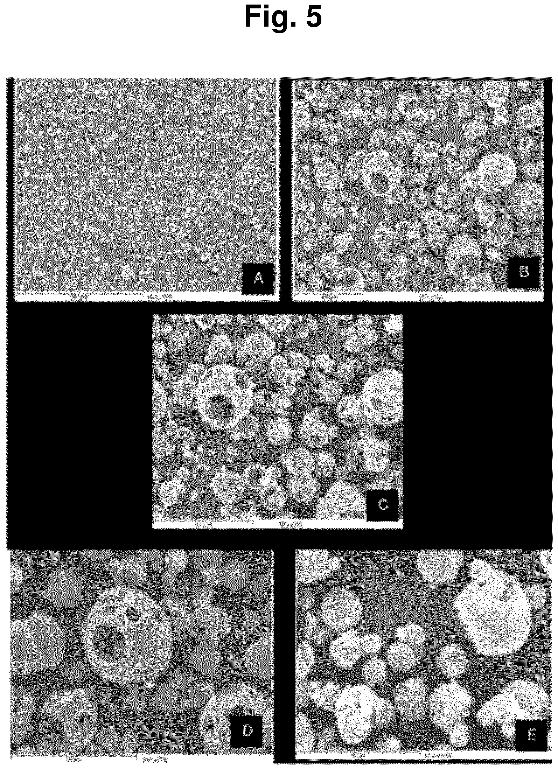

Figure 5 shows the images obtained by means of a molecular scanning electron microscope at different angles of magnification, which allows the detection, processing, and visualization of the signals resulting from the interactions between a high energy electron beam and matter. These interactions provide information on topography, composition, and structure. The image marked with the letter "A" shows a measuring range of 600 micrometers with a degree of magnification of 100x. The image marked with the letter "B" shows a measuring range of 100 micrometers with a degree of magnification of 350x. The image marked with the letter "C" shows a measuring range of 100 micrometers with a degree of magnification of 500x. Image "D" shows a measuring range of 80 micrometers with a degree of magnification of 750x, and the image marked with the letter "E" shows a measuring range of 60 micrometers with a degree of magnification of 1,000x.
   According to the results obtained in the protein sample resulting from the process disclosed in the present description (hereinafter referred to as PROTEO 50 or, P50), it can be observed that there are 2 groups of protein sizes that predominate, which coincides with the electrophoretic analysis and the particle size distribution and zeta potential. These indicate that the protein P50 produced has a homogeneity that allows mixing with other products obtaining a good solubility and stability.

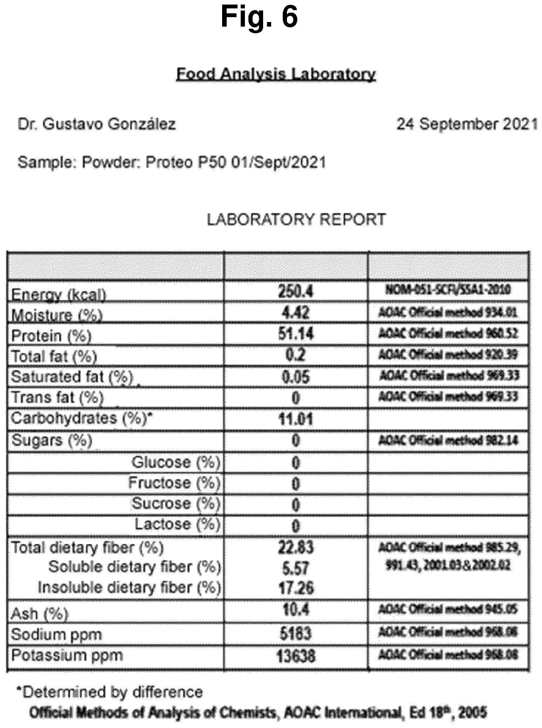

Figure 6 shows a proximate analysis of a sample of protein P50. Among the main results, it is shown that protein P50 contains a good amount of protein represented in nitrogen bonds, as well as an important amount of carbohydrates represented mainly by soluble and insoluble fibers, as well as a very low amount of fats. Protein P50 shows said nutritional advantages, being low in total fat, trans fats, and saturated fats, which are the main causes of cardiovascular diseases. Likewise, due to its high protein content, protein P50 is a viable option to replace red meat, poultry, or fish protein, with the added value that protein P50 does not contribute LDL cholesterol to the diet.

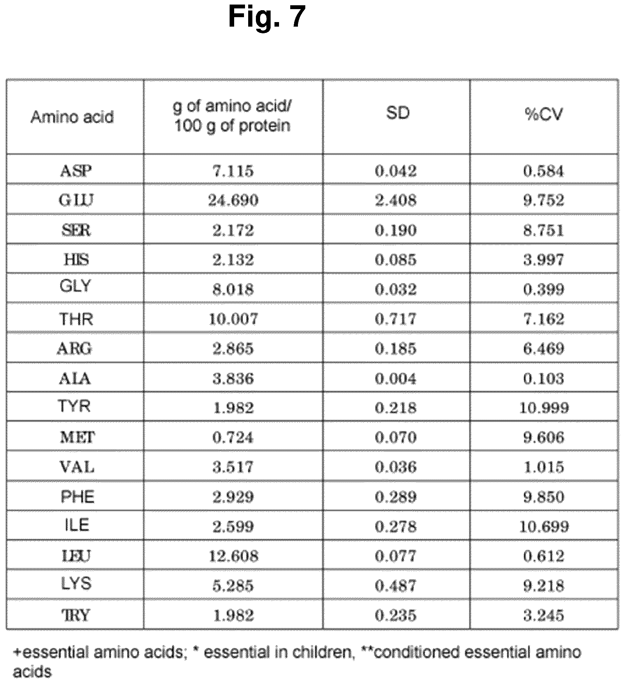

Figure 7 shows the aminogram obtained from a sample of protein P50. The result of the analysis shows that P50 is a complete protein, with a significant amount of all essential and non-essential amino acids included. This is indicative that the addition of protein P50 to various food products can significantly increase the amount of peptides, minerals, and soluble and insoluble fiber, which together could generate various health benefits for the consumer, as well as a possible reduction of spoilage reactions and increased maintenance of muscle mass in adults.

### DETAILED DESCRIPTION OF THE INVENTION

In the last 200 years, and still today, the application of yeasts in winemaking, alcoholic fermentation, and the baking industry is well known.

The first microorganisms used as a protein source were yeasts, particularly *Saccharomyces cerevisiae,* which even today is still the main source of a single-cell protein with a production of 200,000 tons per year in dry weight. *Spirulina maxima, Aspergillus niger, Kluyveromyces fragilis,* and *Candida utilis* are also widely used.

Yeasts are the microorganisms that are the oldest known, best studied, and generally best accepted by consumers. Yeasts are rarely toxic or pathogenic and can be used in food for human consumption. Although their protein content does not exceed 60%, their concentration of essential amino acids such as lysine, tryptophan, and threonine is satisfactory, although their methionine and cysteine content is low. Yeasts are very rich in vitamins (group B) and their nucleic acid content is low, being in the range of 4 to 10%.

The essential amino acid profile is a basic factor when evaluating a protein for human consumption. In the case of yeasts, the concentration of essential amino acids such as lysine, tryptophan, and cysteine is satisfactory, however, they are low in their content of sulfur-containing amino acids such as methionine and cysteine, which is resolved in the concentration process.

Table I, which is inserted below, shows the comparison of amino acids in several products:

**Table I**

| Comparison of amino acid content in several products | | | | | | | | | |
|---|---|---|---|---|---|---|---|---|---|
| Sources | LEU | LYS | SER | THR | VAL | ILE | TRP | MET | MET + CYS |
| SKIM MILK POWDER | 3.41 | 2.70 | 2.10 | 1.60 | 2.40 | 2.20 | 0.49 | 0.86 | 1.37 |
| EGG | 9.00 | 6.70 | 7.70 | 5.30 | 7.20 | 5.80 | - | - | - |
| WHEAT | 6.40 | 2.70 | 4.80 | 2.90 | 4.30 | 3.80 | - | - | - |
| FAO STANDARD (2002) | 7.00 | 5.50 | 4.00 | 4.60 | 5.00 | 4.00 | 1.00 | - | 3.50 |

The single-cell protein is the microbial biomass generated by bacteria, yeasts, or filamentous fungi, cultured under controlled fermentation conditions, which optimize the use of different substrates, whether enriched or not.

The single-cell protein is produced from microbial biomass and in the case of the invention disclosed in the present description, a particular strain of *Kluyveromyces marxianus,* referred to as KMPROTEO 1, was selected, said straining having the following advantages:
1. Rapid growth;
2. Simple and inexpensive culture medium;
3. Simple processing system;
4. Non-pathogenic;
5. Low level of nucleic acids;
6. High nutritional value;
7. Concentration of protein that is in the range of 50 to 60%;
8. Easier to separate.

Conventionally, the single-cell protein has been used successfully in the preparation of products for animal consumption, even in the formulation of replacement milks for livestock growth and fattening. The FDA allows its use in food as flavoring agents, enhancers, and meat extenders. The actual development allows use in various food systems, since the nucleic acid content is very low, given the technology applied for protein purification.

These microorganisms are an inexhaustible source of new nutritional ingredients and additives with excellent functional and nutritional properties. Nowadays, their relevance has increased with the use of innovative preparation and fractionation techniques, mainly provided by biotechnology.

The use of certain microorganisms depends on the substrate, the process, and the desired quality itself of the biomass. Biosafety, technical availability, and biological stability are aspects to be taken into account for selecting a certain strain as a single-cell protein source.

Companies in the food industry are seeking protein sources that are not only inexpensive, but also of high nutritional quality, so the invention disclosed in the terms of the present description is an excellent option since it proposes a solution that is inexpensive, scalable, replicable at an international level and ensures the production of a tested and safe finished product.

The invention disclosed herein presents a solution for optimal use of food; the disclosed invention is based on sustainability and can be successfully replicated.

In general, it has been determined that the protein content in yeast is 45 to 55 %, with the added value of containing a low level of RNA, so it allows its use as a food supplement, both in feed for livestock and in food for human consumption.

The invention disclosed herein relates to a fermentation process to obtain a single-cell biomass; the process enables a high-protein product to be obtained for use in the food industry.

The process disclosed in the present description, specifically in terms of fermentation, handles different parameters to measure different experimental conditions, which allows the process to be directed, the main parameter being the production of biomass in a specific time. Due to the importance in the development of the biomass, a certain strain was chosen, which strain is capable of being developed in a short time, while maintaining a high percentage of substrate to biomass conversion, which is reflected in a higher process yield and, accordingly, in lower production costs.

Despite the fact that there are commercial yeasts for performing fermentations, the use of pure cultures of completely homogeneous yeasts is more effective since it is a process with fewer varying factors and which directly condition the unique characteristic of the products obtained.

Therefore, the selection of the strain suitable for each type of fermentation is a very important strategy to ensure correct fermentation, as well as to improve the characteristics of the end product, since yeasts can produce compounds which slightly distinguish the obtained product, such as the presence or content of glycerol, esters, higher alcohols, etc.

The single-cell protein is produced from microbial biomass and in the case of the invention disclosed herein, it specifically relates to a yeast *Kluyveromyces marxianus,* as it has the following advantages:
1. Rapid growth;
2. Simple and inexpensive culture medium;
3. Simple processing system;
4. Non-pathogenic;
5. Low level of nucleic acids;
6. High nutritional value;
7. Concentration of protein from 50 to 60%; and
8. Easier to separate.

In the specific case of the invention described in the present description, a particular strain of the yeast *Kluyveromyces marxianus* was chosen due to its capacity of high reproduction, production of biomass, and fermentation of carbohydrates, the production of biomass corresponding to 3 g/L/h and a protein content in the finished product being greater than 50%.

The strain used for the process disclosed in the present description is the strain KMPROTEO1, which has been deposited under the terms of the Budapest Treaty on the International Recognition of the Deposit of Microorganisms for the Purposes of Patent Procedure, in the *Centro National de Recursos Genéticos* (CNRG) (National Center for Genetic Resources) of the *Instituto National de Investigaciones Forestales, Agricolas y Pecuarias* (National Institute for Forestry, Agricultural and Livestock Research), with accession number CM-CNRG TB66 (hereinafter, KMPROTEO1), the characteristics of which are described in patent application MX/a/2019/004550, which describes the use for fermenting acid whey to obtain a single-cell biomass, obtaining a high-protein product that is in the range of 50 to 90% for use in the food industry.

Variations in the substrate used for growing the yeast and the fermentation and refining conditions define the composition of the end product.

The process disclosed in the present description relates to a fermentation process, the purpose of which is to obtain single-cell biomass, using a particular strain of *Kluyveromyces marxianus* (yeast KMPROTEO1), which enables a high-protein product to be obtained for use in the food industry.

The finished product resulting from the process disclosed in the terms of the present description, referred to as "PROTEO P50" (or P50), is a powder product, with a light brown color, neutral flavor and smell, which is useful as an additive in food matrices used by the processed food industry. The description of the process uses the term "P50 fermentation medium", which corresponds to the mixture of consumables and substrates required during the fermentation process, which will be absorbed by the yeast to generate a single-cell biomass.

### Process

The production process for producing the biomass comprises the following steps:
1. Reactivating yeast strain KMPROTEO1 in a Petri dish using YPD agar for 48 hours at a temperature of between 35°C and 38°C;
2. Inoculating from the Petri dish to 20 ml liquid medium using 4 tubes with 5 ml of medium in suspension with an optical density of 1.0, maintaining the inoculum between 35° and 38°C for 4 to 8 hours;
3. Transferring the culture from the preceding step to a flask with "P50 fermentation medium";

| **P50 fermentation medium** | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |

being left to ferment for 4 to 8 hours maintaining a temperature of 35°C to 38°C with stirring between 100 and 250 revolutions per minute (rpm) with a pH between 5 and 5.5 and an aeration level between 0.5 and 2 volumes of air/volume of medium per minute (wm);
4. Transferring the culture from the preceding step to a seed reactor which contains the "P50 fermentation medium", being left to ferment for 4 to 8 hours maintaining a temperature of 35°C to 38°C with stirring between 100 and 250 rpm with a pH between 5 and 5.5 and an aeration level between 0.5 and 2 volumes of air/volume of medium per minute (vvm);
5. Transferring the culture from the preceding step to a production reactor which contains the "P50 fermentation medium", being left to ferment for 12 to 23 hours maintaining a temperature of 35°C to 38°C with stirring between 100 and 250 rpm with a pH between 5 and 5.5 and an aeration level between 0.5 and 2 volumes of air/volume of medium per minute (vvm), maintaining the concentration of dextrose in the medium between 1 a 2 grams per liter;
6. Centrifuging the mixture at 6,000 rpm, separating the mixture into two phases, pumping the light phase to a tank and the heavy phase to a second tank;
7. Re-suspending the heavy phase in the same amount of removed water, centrifuging again following the conditions of the preceding step, performing this process twice;
8. Re-suspending the heavy phase in the reaction tank, adjusting the percentage of solids to 10%;
9. Adding the enzyme cellulase in a proportion of between 0.1% and 0.2% of wet biomass, maintaining a temperature between 55°C and 60°C for between 6 and 12 hours with stirring between 100 and 250 rpm, maintaining the pH between 5 and 5.5, and where the pH is adjusted to 7 when this step is completed;
10. Raising the temperature between 65°C and 75°C and maintaining it for 15 to 30 minutes;
11. Evaporating the mixture until concentrating the solids within the operating range of between 20% and 30% of solids in the mixture;
12. Drying in a spray dryer at an inlet temperature between 185°C and 195°C and an outlet temperature between 70°C and 80°C;
13. Recovering the powder from the dryer and packaging the end product in a food-grade bag.

The following example allows showing the characteristics of the product obtained using the process object of the present application:

### Example

1. Reactivating the strain of yeast *Kluyveromyces Marxianus* in a Petri dish using YPD agar for 48 hours at a temperature of 38°Centigrade,
2. Inoculating from the Petri dish to 20 milliliters (ml) liquid medium using 4 tubes with 5 milliliters (ml) of medium in suspension with an optical density of 1.0, maintaining the inoculum at 38°C for 8 hours;
3. Transferring the 20 ml of inoculum to a flask with 180 ml with the "P50 medium", fermenting for 8 hours maintaining a temperature of 38°C with stirring at 150 revolutions per minute (rpm) with a pH between 5 and 5.5 and an aeration level of 1 volume of air/volume of medium per minute (vvm);
4. Transferring the 200 ml of the fermentation from the preceding step to a flask with 3.8 liters of the "P50 medium", fermenting for 8 hours maintaining a temperature of 38°C with stirring at 150 revolutions per minute (rpm) with a pH between 5 and 5.5 and an aeration level of 1 volume of air/volume of medium per minute (wm);
5. Transferring the 4 liters of the preceding inoculum to the seed reactor containing 36 liters of the "P50 medium", fermenting for 8 hours maintaining a temperature of 38°C with stirring at 150 revolutions per minute (rpm) with a pH between 5 and 5.5 and an aeration level of 1 volume of air/volume of medium per minute (wm);
6. Transferring the 40 liters from the seed reactor to the production reactor containing 160 liters of the "P50 medium", fermenting for 16 hours maintaining a temperature of 38°C with stirring at 150 revolutions per minute (rpm) with a pH between 5 and 5.5 and an aeration level of 1 volume of air/volume of medium per minute (vvm), maintaining the concentration of dextrose in the medium between 1 gram per liter and 2 grams per liter, where the recovered product in dry weight is equivalent to 6 kg of protein P50;
7. Centrifuging the mixture at 6,000 revolutions per minute (rpm), separating the mixture into two phases, pumping the light phase to a tank and the heavy phase to a second tank;
8. Re-suspending the heavy phase in the same amount of removed water, centrifuging again following the specifications of step 7, and performing this process twice;
9. Re-suspending the heavy phase in the reaction tank, adjusting the percentage of solids to 10%;
10. Adding the enzyme cellulase at a proportion of 0.2% of wet biomass, maintaining a temperature of 60°C for at least 12 hours with stirring at 150 revolutions per minute (rpm), maintaining the pH between 5 and 5.5, where the pH is adjusted to 7 when the autolysis process is completed;
11. Raising the temperature to 75°C and maintaining it for 15 minutes to inactivate the enzymes;
12. Evaporating the mixture until concentrating the solids within the operating range of between 20% and 30% of solids in the mixture;
13. Drying in a spray dryer at an inlet of 185°C and an outlet of 80°C;
14. Recovering the powder from the dryer and packaging it in a food-grade bag.

The protein which is obtained with the disclosed process, this process is a clear, water-dispersible, and tasteless beige powder that has no smell, such that it does not change the organoleptic properties of the products to which it is added as a food additive.

These characteristics allow the ingredient to be added to food products without significantly changing the organoleptic or rheological properties.

Table II inserted below itemizes the nutrimental characterization of protein P50.

**Table II**

| Nutrimental characterization of protein p50 | | |
|---|---|---|
| PARAMETER | VALUE | METHODOLOGY |
| Energy (kcal) | 250.4 | NOM-051-SCFI/SSA1-2010 |

| PARAMETER | VALUE | METHODOLOGY |
|---|---|---|
| Moisture (%) | 4.42 | AOAC Official method 934.01 |
| Protein (%) | 49.89 | AOAC Official method 960.52 |
| Total fat (%) | 0.2 | AOAC Official method 920.39 |
| Saturated fat (%) | 0.05 | AOAC Official method 969.33 |
| Trans fat (%) | 0 | AOAC Official method 969.33 |
| Carbohydrates (%) * | 11.01 | |
| Sugars (%) | 0 | AOAC Official method 982.14 |
| Glucose (%) | 0 | |
| Fructose (%) | 0 | |
| Sucrose (%) | 0 | |
| Lactose (%) | 0 | |
| Total dietary fiber (%) | 22.83 | AOAC Official method 985.29, 991.43, 2001.03 and 2002.02 |
| Ash (%) | 10.4 | AOAC Official method 945.05 |

One way of proving the effectiveness of the disclosed process and the advantages in terms of the characteristics of the product obtained from it is by comparing it with other protein sources. The comparison of the nutrimental characterization of the protein obtained by the process disclosed in the present description compared with two other proteins of animal origin is shown in Table III, which is inserted below:

**Table III**

| Nutrimental characterization: amino acids | | | |
|---|---|---|---|
| Amino acid | Protein | | |
| | PROTEO P50 | Fish | Beef |
| ILE⁺ | 2.599 | 3.00 | 5.1 |
| LEU⁺ | 12.608 | 4.50 | 8.4 |
| LYS⁺ | 5.285 | 5.00 | 8.4 |
| MET⁺ | 0.724 | 1.10 | 2.3 |
| PHE⁺ | 2.929 | 2.15 | 4.0 |
| THR⁺ | 10.007 | 2.50 | 4.0 |

| Amino acid | Protein | | |
|---|---|---|---|
| | PROTEO P50 | Fish | Beef |
| VAL⁺ | 3.517 | 3.10 | 5.7 |
| HIS* | 2.132 | 1.50 | 2.9 |
| ARG** | 2.865 | 2.80 | 6.6 |
| GLU** | 24.690 | 4.00 | 14.4 |
| GLY** | 8.018 | - | 2.9 |
| TYR** | 1.982 | 2.00 | 1.1 |
| SER | 2.172 | 2.10 | 3.8 |
| ALA | 3.836 | 4.00 | 6.4 |
| ASP | 7.115 | 6.00 | 8.8 |
| protein (%) | 53.14 | 16-22 | 23 |

Additionally, the nutrimental characterization (minerals) of three batches of protein P50 obtained by the process disclosed herein was performed, determining the compositions shown in Table IV, which is inserted below:

**Table IV**

| Nutrimental characterization: minerals | | | | | | |
|---|---|---|---|---|---|---|
| Minerals mg/100 g sample | Cu | Fe | Mn | Ni | Zn | Cr |
| Prot-A1 | 2.64 | 2.16 | 0.126 | 0.132 | 11.65 | 0.03518 |
| Prot-B1 | 2.52 | 2.35 | 0.149 | 0.137 | 12.51 | 0.03150 |
| Prot-C1 | 2.47 | 2.89 | 0.147 | 0.140 | 11.798 | 0.03474 |
| EAR (adults) | 900 µg/day | 8-18 mg/day | 1.8-2.3 mg/day | IDA 5 ug/kg/day | 8-11 mg/day | 25-35 µg/day |

| | | | | | | |
|---|---|---|---|---|---|---|
| *The names A1, B1, and C1 refer to 3 batches of protein P50. | | | | | | |

Additionally, for the purpose of proving the harmlessness of the product obtained by the process disclosed in the present description, the microbiological characterization has been performed and is shown in Table V, which is inserted below:

**Table V**

| No. | Description | Specification | |
|---|---|---|---|
| 1 | Total bacteria count | ≤1,000 CFU/g | |
| 2 | Yeast and molds | ≤100 CFU/g | |
| 3 | Total coliforms | <10 CFU/g | |
| 4 | *Salmonella* spp | Negative in 25 g | |
| 5 | *Staphylococcus aureus* | Negative in 10 g | |

Some of the advantages of protein P50 obtained from the process disclosed in the present description are: i) its low fat content, with a high nutritional value (wide range of amino acids); ii) its high functionality for use as an additive, having a low handling and storage cost; iii) its long shelf-life (greater than 24 meses), and; iv) it is produced with a low production time and cost.

Some other additional advantages are:
(i) The protein is produced by means of controlled fermentation in a plant with Good Manufacturing Practice certification (GMP by the FDA);
(ii) The substrates used as a growth medium for the microorganism are food grade;
(iii) The process disclosed allows it to be performed in a basic infrastructure and with a high capacity to provide protein in large quantities;
(iv) The characteristics of the product prepared with the disclosed process has positive physiological effects.

Some embodiments of the invention disclosed in the present description will be described below by way of example, showing some of the possible uses that the product resulting from the disclosed fermentation process may have.

The description of some of the preferred methods of use of the present invention which are exemplified below does not intend to limit the scopes thereof, since the multiple uses of the protein resulting from the production and formulation process of a single-cell protein described in the present description will be apparent for a person having ordinary skill in the art.

A battery of tests were performed to demonstrate that the single-cell protein PROTEO P50, disclosed in the present description, can be used in the formulation of food typically containing products and protein of animal origin.

The food products in which the addition of PROTEO P50 disclosed in the present description was evaluated were sausages, hamburger meat, and hamburger bun. They were all prepared with products of plant origin, replacing components such as egg, milk, and meat with vegetables, legumes, grains, and beverages of plant origin and admixed with single-cell yeast protein PROTEO P50.

The sausages were prepared with two different formulations, the first using rice, cassava starch, corn starch, and oat as a base; paprika powder, salt, garlic powder, and liquid smoke as seasonings; vegetable oil as a substitute for animal fat and water. The second formulation used potato, carrot, and beetroot as a base, as well as paprika powder, salt, garlic powder, and liquid smoke as seasonings; and vegetable oil was used as a substitute for animal fat and water. The single-cell yeast protein PROTEO P50 disclosed in the present description was added to both formulations.

The different hamburger meat formulations contain a mixture of legumes (lentils, chickpeas, and beans) in different proportions; rice to simulate fat in the meat, cassava starch and oat meal as binding agents; vegetable oil as a substitute for animal fat, beetroot paste as a colorant, and a mixture of spices (salt, cummin, garlic powder, paprika powder) to simulate the flavor of hamburger meat.

The hamburger bun was prepared with white flour, vegetable oil, standard sugar, salt, white vinegar, sodium bicarbonate, yeast, warm water, and plant-based milk.

### Examples

### Vegan sausages

In one test, different formulations of mixtures admixed with protein PROTEO P50, disclosed in the present description were evaluated, selecting those that had a color and texture similar to conventional sausages, obtaining 2 formulations which were referred to as:
Control 1 + PROTEO P50 and its control 1 (Table VI) and,
Control 2 + PROTEO P50 and its respective control 2 (Table VII).

**Table VI**

| Sausage formulation of control 1 + control 1 + PROTEO P50 | | |
|---|---|---|
| Ingredients | Control 1 | Control 1 + PROTEO P50 |
| Rice paste | 58.06 % | 49.28 % |
| Garlic powder | 0.49% | 0.42 % |
| Cassava starch | 6.23 % | 5.29 % |
| Corn starch | 2.62 % | 2.23 % |
| Paprika powder | 0.98 % | 0.84 % |
| Vegetable oil | 5.9 % | 9.28 % |
| Salt | 1.48 % | 1.25 % |
| Beetroot paste | 4.92 % | 6.68 % |
| Liquid smoke | 0.16 % | 0.14 % |
| Oat meal | 2.73 % | 2.32 % |
| Water | 16.43 % | 13.92 % |
| PROTEO P50 | 0.00 | 8.48 % |

**Table VII**

| Control 2 + control 2 + PROTEO P50 sausage formulation | | |
|---|---|---|
| Ingredients | Control 2 | Control 2 + PROTEO P50 |
| Potato paste | 46.35 % | 42.41 % |
| Carrot paste | 7.72 % | 7.07 % |
| Beetroot paste | 15.45 % | 14.14 % |
| Oat meal | 23.17 % | 21.08 % |
| Oil | 3.86 % | 3.53 % |
| Salt | 2.16 % | 1.98 % |
| Garlic powder | 0.46 % | 0.42 % |
| Pepper powder | 0.15 % | 0.14 % |
| Paprika powder | 0.46 % | 0.42 % |
| Cummin | 0.05 % | 0.05 % |
| Liquid smoke | 0.15 % | 0.15 % |
| PROTEO P50 | 0.00 % | 8.48 % |

### Raw materials

The vegetables and condiments (Table VII) were acquired at a local market in Hermosillo, Sonora, and were stored at 4°C until the time they were used.

### Weighing of ingredients

This was carried out as established in Tables VI and VII using an analytical balance.

### Grinding and mixing

This step was performed with the help of a Moulinex^{®} mixer to favor continuous mixing. The ingredients were added in the following order: vegetable pastes, oat meal, salt, condiments, and protein PROTEO P50 (in the case of formulations with protein, liquid smoke), and lastly, the rice paste or potato paste, which acted as binders for the mixture to remain compact, depending on the formulation.

### Stuffing

For stuffing, a biscuit-type equipment (Betterware^{®} brand) was used, using artificial casing. 8 cm sausages were formed (removing air bubbles in each sausage), which were blanched in water at 90°C for 30 min. Subsequently, thermal shocking was performed with cold water, and they were removed from the water and left to stand for 30 min.

### Storing

Once the sausages were obtained, they were kept cooled at 4°C, obtaining a vegan-type sausage as the end product.

### Sensory evaluation

Acceptance by consumers (6 people) was evaluated based on the characteristics of smell, color, flavor, and texture, using a hedonic scale with the following descriptors: Bad = 1, Fair = 2, Good = 3 and Excellent = 4.

The sausages were cut into 2.5 cm portions. Each consumer was given a portion of each formulation to try.

### Results

A sausage-type product with a firm texture and which does not adhere to the artificial casing, with no strange smells or flavors, was obtained.

Table VIII shows the results of the sensory evaluation based on the characteristics of smell, flavor, color and texture.

The Control 1 + PROTEO P50 and Control 2 + PROTEO P50 formulations exhibited differences in terms of color and flavor.

The panelists mentioned that Control 2 + PROTEO P50 has a residual flavor or aftertaste of carrot.

Control 2 + PROTEO P50 was the most widely accepted in terms of the color parameter.

Control 1 + PROTEO P50 was the most widely accepted in terms of the flavor and smell parameters.

For the texture parameter, no difference was found between sausage of Control 1 + PROTEO P50 and sausage of Control 2 + PROTEO P50.

**Table VIII**

| Sensory evaluation of sausages | | | | |
|---|---|---|---|---|
| Sausage | Smell | Color | Flavor | Texture |
| Control 1 | 3.8 | 3.4 | 3.4 | 3.7 |
| Control 1 + PROTEO P50 | 3.9 | 3.4 | 3.9 | 3.9 |
| Control 2 | 4 | 3.8 | 3.6 | 3.7 |
| Control 2 + PROTEO P50 | 3.8 | 3.9 | 3.5 | 3.8 |

### Preparation of hamburger

Two different formulations referred to as Formulation 7 and Formulation 5, and their controls (without PROTEO P50), were prepared.

Formulation 5 was prepared with bean paste (Table IX) and formulation 7 was prepared with lentil paste (Table X), as a base for replacing products of animal origin.

**Table IX**

| Hamburger formulations | | | | |
|---|---|---|---|---|
| Ingredient / formulation | Formulation 5 | Formulation 5 with PROTEO P50 | Formulation 7 | Formulation 7 with PROTEO P50 |
| Vegetable oil | 6.87 % | 6.2 % | 6.9 % | 6.2 % |
| Purified water | 16.57 % | 15.7 % | 17.5 % | 15.6 % |
| Garlic powder | 0.10% | 0.1% | 0.1% | 0.1% |
| Rice paste | 8.59% | 7.8 % | 8.6 % | 7.8 % |
| Beetroot paste | 4.29 % | 3.9 % | 4.3 % | 3.9 % |
| Cummin powder | 0.10% | 0.1% | 0.1% | 0.1% |
| Corn starch | 6.01 % | 5.5 % | 6.0 % | 5.5 % |
| Black bean paste | 17.80 % | 15.6 % | 0.0 % | 0.00 % |
| Chickpea paste | 25.76 % | 23.4 % | 25.8 % | 23.4 % |
| Oat meal | 12.02 % | 10.9 % | 12 % | 10.1% |
| Meat-flavored smoke | 0.00 % | 0.00 % | 0.00 % | 0.00 % |
| Lentil paste | 0.00 % | 0.00 % | 16.8 % | 16.5 % |
| Paprika powder | 1.03 % | 0.9 % | 1.0 % | 0.9 % |
| PROTEOP50 | 0.00 % | 9.1 % | 0.00 % | 9.1 % |
| Salt | 0.86 % | 0.8 % | 0.9 % | 0.8 % |

### Raw materials

The vegetables and condiments were acquired at a local market in Hermosillo, Sonora, and were stored at 4°C until the time they were used.

### Weighing of ingredients

This was performed using an analytical balance.

### Grinding and mixing

These steps were performed with the help of a Moulinex^{®} mixer to favor continuous mixing. The ingredients were added in the following order: bean and/or chickpea pastes, oat meal, salt, condiments, and protein PROTEO P50 disclosed in the present description, in the case of formulations with protein. The rice paste, which acted as a binder for the mixture to remain compact, was subsequently added. Lastly, the beetroot paste was incorporated to impart a color similar to that of meat.

### Weighing

Several quantities of the mixture described above were taken and weighed in 25 g portions using an analytical balance.

### Forming and packing

In this step, a circular plastic former was used for each portion of hamburger meat, and they were wrapped in high density casings.

### Storing

Once the formed hamburgers were obtained, they were kept frozen at a temperature of -10°C to obtain a vegan-type hamburger meat as an end product.

### Sensory evaluation

Acceptance by consumers (6 people) was evaluated based on the characteristics of smell, color, and texture, using a hedonic scale with the following descriptors:
Bad = 1, Fair = 2, Good = 3 and Excellent = 4.

Each consumer was asked to evaluate a disc-shaped portion of 10 g of vegan hamburger meat of each formulation.

### Results

A vegan hamburger meat-type product having a pleasant color and smell was obtained as a result.

Table X shows the results of the sensory evaluation based on the characteristics of smell, color, and texture. The 4 formulations exhibited no differences in terms of color and flavor.

**Table X**

| Sensory evaluation of vegan hamburger meat | | | |
|---|---|---|---|
| Formulation | Smell | Color | Texture |
| Formulation 5 | 3.7 | 3.8 | 3.9 |
| Formulation 5 with PROTEOP50 | 3.6 | 3.8 | 3.0 |
| Formulation 7 | 3.7 | 3.7 | 3.8 |
| Formulation 7 with PROTEOP50 | 3.7 | 3.7 | 2.9 |

The panelists mentioned that the texture of Formulation 5, prepared with protein PROTEO P50 disclosed in the present description, and Formulation 7 with PROTEO P50, is not similar to that of a conventional hamburger. The panelists recommended improving the texture of the formulations with the protein.

### Hamburger bun

In preliminary tests, different formulations of vegan hamburger buns and mixtures admixed with the protein PROTEO50, disclosed in the present description, were evaluated, selecting that which exhibited characteristics of flavor, color, and smell of a commercial hamburger bun (Table XI).

**Table XI**

| Hamburger bun formulation | | |
|---|---|---|
| Ingredients | Formulation with PROTEO P50 | Control (without PROTEO P50) |
| Warm water | 7.90 % | 7.90 % |
| Yeast | 1.30 % | 1.30 % |
| White flour | 44.50 % | 45.30 % |
| Vegetable oil | 8.50 % | 8.50 % |
| Standard sugar | 5.20 % | 5.20 % |
| Salt | 0.80 % | 0.80 % |
| Vinegar | 0.20 % | 0.20 % |
| Sodium bicarbonate | 0.10 % | 0.10 % |
| PROTEO P50 | 7.90 % | 0.00 % |
| Plant-based milk | 23.60 % | 23.60 % |

### Raw materials

To prepare the bun, the raw materials were adapted to the preparation of a conventional hamburger bun. The raw materials were acquired from a local market in Hermosillo, Sonora, and were stored at 4°C until the time they were used.

### Weighing of ingredients

This was carried out as established in Table XI using an analytical balance.

### Fermentation 1

The yeast, sugar, and warm water were mixed in a vessel, and the mixture was left to stand for 10 min.

### Mixing

This step was performed in a Moulinex ^{®} mixer. The ingredients and fermentation 1 were mixed in a Moulinex^{®} mixer at a low speed for a time of 10 to 15 min.

### Fermentation 2

Once all the ingredients were mixed together, they were left to stand at room temperature for 30 min.

### Rolling

After the 30 min, manual rolling was performed to homogenize all the components and to finish imparting consistency to the product for 5 min.

### Cutting and weighing

The rolled dough was cut to facilitate weighing (60 g a piece).

### Kneading-forming

This was performed by hand to impart the bun shape to the portions of dough obtained from weighing. These were kneaded, imparting to them a semi-elongated shape, which was completed and the size of the roll was subsequently defined.

### Fermentation 3

Once kneaded, the rolls were left to stand at room temperature for 30 min.

### Cooking

This was performed at a temperature of 190°C for a time of 13 min in a T-Fal^{®} brand toaster oven.

### Cooling

The buns were cooled at room temperature for 40 min, subsequently being cooled until the analysis is performed.

### Sensory evaluation

Acceptance by consumers (10 people) was evaluated based on the characteristics of smell, color, flavor, and texture, using a hedonic scale with the following descriptors: Bad = 1, Fair = 2, Good = 3 and Excellent = 4.

Consumers were given a small piece (20 g) of hamburger bun at a temperature of 30°C. Each consumer was given the sample referred to as CONTROL and the sample with the formulation with protein PROTEO 50 disclosed in the present description to try.

### Results

A product having a pleasant flavor, color, and smell of a vegan hamburger-type bun was obtained.

Table XII shows the results of the sensory evaluation based on the characteristics of smell, flavor, color, and texture.

**Table XII**

| Sensory evaluation of hamburger bun | | | | |
|---|---|---|---|---|
| Sausage | Smell | Color | Flavor | Texture |
| Formulation with PROTEO 50 | 3.9 | 3.9 | 3.8 | 3.4 |
| Control | 4.0 | 3.8 | 3.9 | 3.9 |

In general, consumers did not report any differences in the smell, color, and flavor of the bread admixed with protein PROTEO50 disclosed in the present description and the control.

### Industrial applicability

The fermentation process for the production of single-cell biomass disclosed in the present description, which enables a high-protein product to be obtained, using dextrose and other nutrients as a propagation medium for use by *Kluyveromyces marxianus* to obtain a single-cell biomass and, subsequently, a single-cell protein, and the product obtained directly from the process are concepts which clearly comply with the requirement of industrial applicability.

It should be noted that as of this date, the best method known by the applicant for carrying out to practice the mentioned invention is the one that is apparent from the reading of the present description.

Although the invention has been described in certain detail by way of illustration and with examples for the sake of clarity of understanding, it will become rapidly apparent for those having ordinary skill in the art, in light of the teachings of this invention, that changes and modifications may be made to same without departing from the spirit or scope of the description herein, including the attached claims.

## Claims

1. A process to obtain a single-cell protein using a strain of *Kluyveromyces marxianus* (KMPROTEO1), which has been deposited under the terms of the Budapest Treaty, with accession number CM-CNRG TB66, **characterized in that** it comprises the following steps:
(i) Reactivating yeast strain KMPROTEO1 in a Petri dish using YPD agar for 48 hours at a temperature of between 35°C and 38°C;
(ii) Inoculating from the Petri dish to 20 ml liquid medium using 4 tubes with 5 ml of medium in suspension with an optical density of 1.0, maintaining the inoculum between 35°C and 38°C for 4 to 8 hours;
(iii) Transferring the culture from the preceding step to a flask with P50 medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 4 to 8 hours maintaining a temperature of 35°C to 38°C with stirring between 100 and 250 rpm, with a pH between 5 and 5.5 and an aeration level between 0.5 and 2 volumes of air/volume of medium per minute (wm);
(iv) Transferring the culture from the preceding step to a flask with P50 medium comprising:
| P50 fermentation medium | |
|---|---|
| Dextrose | 30 g/L |
| | |
|---|---|
| Ammonium sulfate | 2 g/L |
| Magnesium sulfate | 0.5 g/L |
| Monopotassium phosphate | 1.5 g/L |
| Yeast extract | 2.5 g/L |
fermenting for 4 to 8 hours maintaining a temperature of between 35°C and 38°C with stirring between 100 and 250 rpm with a pH between 5 and 5.5 and an aeration level between 0.5 and 2 vvm;
(v) Transferring the culture from the preceding step to the seed reactor which contains the P50 medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 4 to 8 hours maintaining a temperature of 35°C to 38°C with stirring between 100 and 250 rpm with a pH between 5 and 5.5 and an aeration level between 0.5 and 2 vvm;
(vi) Transferring the culture from the preceding step to the production reactor which contains the medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 12 to 23 hours maintaining a temperature of 35°C to 38°C with stirring between 100 and 250 rpm with a pH between 5 and 5.5 and an aeration level between 0.5 and 2 (wm), maintaining the concentration of dextrose in the medium between 1 g/L and 2 g/L;
(vii) Centrifuging the mixture at 6,000 rpm, separating the mixture into two phases, pumping the light phase to a tank and the heavy phase to a second tank;
(viii) Re-suspending the heavy phase in the same amount of removed water, centrifuging again following the specifications of the preceding step, and performing this process twice;
(ix) Re-suspending the heavy phase in the reaction tank and adjusting the percentage of solids to 10%;
(x) Adding the enzyme cellulase in a proportion of between 0.1% and 0.2% of wet biomass, maintaining a temperature between 55°C and 60°C for no less than 6 hours and no more than 12 hours with stirring between 100 and 250 rpm, maintaining the pH between 5 and 5.5, where the pH is adjusted to 7 when this step is completed;
(xi) Raising the temperature between 65°C and 75°C and maintaining it for 15 to 30 minutes;
(xii) Evaporating the mixture until concentrating the solids within the operating range of between 20% and 30% of solids in the mixture;
(xiii) Drying in a spray dryer at an inlet temperature between 185°C and 195°C and an outlet temperature between 70°C and 80°C;
(xiv)Recovering the powder from the dryer and packaging it in food-grade bags.

2. A process to obtain a single-cell protein using a strain of *Kluyveromyces marxianus* (KMPROTEO1), which has been deposited under the terms of the Budapest Treaty, with accession number CM-CNRG TB66, **characterized in that** it comprises the following steps:
(i) Reactivating yeast strain KMPROTEO1 in a Petri dish using YPD agar for 48 hours at a temperature of 38°C;
(ii) Inoculating from the Petri dish to 20 ml liquid medium using 4 tubes with 5 ml of medium in suspension with an optical density of 1.0, maintaining the inoculum at a temperature of 38°C for 4 hours;
(iii) Transferring the culture from the preceding step to a flask with P50 medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 4 hours maintaining a temperature of 38°C with stirring at 150 rpm, with a pH of 5 and an aeration level of 1 volume of air/volume of medium per minute (vvm) ;
(iv) Transferring the culture from the preceding step to a flask with P50 medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 4 hours maintaining a temperature of 38°C with stirring at 150 rpm, with a pH of 5 and an aeration level of 1 volume of air/volume of medium per minute (vvm) ;
(v) Transferring the culture from the preceding step to the seed reactor which contains the P50 medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 4 hours maintaining a temperature of 38°C with stirring at 150 rpm, with a pH of 5 and an aeration level of 1 volume of air/volume of medium per minute (vvm) ;
(vi) Transferring the culture from the preceding step to the production reactor which contains the medium comprising:
| P50 fermentation medium | |
|---|---|
| Dextrose | 30 g/L |
| Ammonium sulfate | 2 g/L |
| Magnesium sulfate | 0.5 g/L |
| Monopotassium phosphate | 1.5 g/L |
| Yeast extract | 2.5 g/L |
fermenting for 16 hours maintaining a temperature of 38°C with stirring at 150 rpm with a pH of 5 and an aeration level of 1 (wm), maintaining the concentration of residual dextrose in the medium between 1 g/L and 2 g/L;
(vii) Centrifuging the mixture at 6,000 rpm, separating the mixture into two phases, pumping the light phase to a tank and the heavy phase to a second tank;
(viii) Re-suspending the heavy phase in the same amount of removed water, centrifuging again following the specifications of the preceding step, and performing this process twice;
(ix) Re-suspending the heavy phase in the reaction tank and adjusting the percentage of solids to 10%;
(x) Adding the enzyme cellulase in a proportion of 0.1% v/v of wet biomass, maintaining a temperature of 55°C for 8 hours with stirring at 150 rpm, maintaining the pH between 5 and 5.5, where the pH is adjusted to 7 when this step is completed;
(xi) Raising the temperature to 75°C and maintaining it for 15 minutes;
(xii) Evaporating the mixture until concentrating the solids within the operating range of between 20% and 30% of solids in the mixture;
(xiii) Drying in a spray dryer at an inlet temperature of 185°C and an outlet temperature of 70°C;
(xiv)Recovering the powder from the dryer and packaging it in food-grade bags.

3. A protein product which is obtained directly from the process according to claim 1, **characterized in that** the nutrimental identification thereof is:
| PARAMETER | VALUE |
|---|---|
| Energy (kcal) | approx. 250.4 |
| Moisture (%) | approx. 4.42 |
| Protein (%) | approx. 49.89 |
| Total fat (%) | approx. 0.2 |
| Saturated fat (%) | approx. 0.05 |
| Trans fat (%) | 0 |
| Carbohydrates (%) * | approx. 11.01 |
| Sugars (%) | 0 |
| Glucose (%) | 0 |
| Fructose (%) | 0 |
| Sucrose (%) | 0 |
| Lactose (%) | 0 |
| Total dietary fiber (%) | approx. 22.83 |
| Ash (%) | approx. 10.4 |

4. A protein product which is obtained directly from the process according to claim 1, **characterized in that** the amino acid profile thereof is:
| Amino acid | PROTEO 50 |
|---|---|
| ILE⁺ | approx. 2.599 |
| LEU⁺ | approx. 12.608 |
| LYS⁺ | approx. 5.285 |
| MET⁺ | approx. 0.724 |
| PHE⁺ | approx. 2.929 |
| THR⁺ | approx. 10.007 |
| VAL⁺ | approx. 3.517 |
| HIS* | approx. 2.132 |
| ARG** | approx. 2.865 |
| GLU** | approx. 24.690 |
| GLY** | approx. 8.018 |
| TYR** | approx. 1.982 |
| SER | approx. 2.172 |
| ALA | approx. 3.836 |
| ASP | approx. 7.115 |
| protein (%) | approx. 53.14 |

5. A protein product which is obtained directly from the process according to claim 1, **characterized in that** the mineral supply thereof is:
| **Mineral mg/100 g sample** | **Content** |
|---|---|
| Cu | approx. 2.47 to 2.64 |
| Fe | approx. 2.16 to 2.89 |
| Mn | approx. 0.126 to 0.149 |
| Ni | approx. 0.140 to 0.132 |
| Zn | approx. 11.65 to 12.51 |
| Cr | approx. 0.03474 to 0.03518 |

6. The protein product according to claims 3 to 5, **characterized in that** it is used as a flour food supplement or as an additive for meat products.

7. The protein product according to claim 6, **characterized in that** it is used as a flour food supplement.

8. The protein product according to claim 6, **characterized in that** it is used as an additive for meat products.
